# EUROPÄISCHE PATENTSCHRIFT

(11) **EP 1 863 790 B1**
(45) Veröffentlichungstag und Bekanntmachung des Hinweises auf die Patenterteilung: **10.02.2010**
(21) Anmeldenummer: 06723257.9
(22) Anmeldetag: 07.03.2006
(51) Int. Cl.: C07D 401/04, A61K 31/435, A61P 3/10

(54) **AMID SUBSTITUIERTE 8-N-BENZIMIDAZOLE, VERFAHREN ZU IHRER HERSTELLUNG UND IHRE VERWENDUNG ALS ARZNEIMITTEL**
AMIDE-SUBSTITUTED 8-N-BENZIMIDAZOLES, METHOD FOR THE PRODUCTION THEREOF, AND USE OF THE SAME AS MEDICAMENTS
8-N-BENZIMIDAZOLES A SUBSTITUTION AMIDE, LEUR PROCEDE DE PRODUCTION, ET LEUR UTILISATION EN TANT QUE MEDICAMENTS

(30) Priorität: 19.03.2005 DE 102005012874
(43) Veröffentlichungstag der Anmeldung: 12.12.2007
(73) Patentinhaber: Sanofi-Aventis, 75013 Paris (FR)
(72) Erfinder: DEFOSSA, Elizabeth, 65926 Frankfurt am Main (DE); SCHOENAFINGER, Karl, 65926 Frankfurt am Main (DE); JAEHNE, Gerhard, 65926 Frankfurt am Main (DE); BUNNING, Christian, 65926 Frankfurt am Main (DE); TSCHANK, Georg, 65926 Frankfurt am Main (DE); WERNER, Ulrich, 65926 Frankfurt am Main (DE)
(74) Vertreter: Fischer, Hans-Jürgen
(86) Internationale Anmeldenummer: PCT/EP2006/002058
(87) Internationale Veröffentlichungsnummer: WO 2006/099943

(56) Entgegenhaltungen:
- EP-A- 1 069 124
- WO-A-02/48152
- WO-A-03/104233

## Beschreibung

Die Erfindung betrifft Amid substituierte 8-N-Benzimidazole sowie deren physiologisch verträgliche Salze und physiologisch funktionelle Derivate.

EP 1069124 beschreibt 2-Benzimdazolylamine als ORL-1 Rezeptor Agonisten.
WO 97/12615 beschreibt Benzimidazolderivate als 15-LO Inhibitoren.
WO 02/04425 beschreibt strukturähnliche Virus Polymerase Inhibitoren.
WO 02/46168 beschreibt Benzimidazolderivate zur Behandlung der Alzheimer Krankheit.

Der Erfindung lag die Aufgabe zugrunde, Verbindungen zur Verfügung zu stellen, die eine therapeutisch verwertbare Blutzucker senkende Wirkung entfalten. Diese Verbindungen sollen sich insbesonders zur Behandlung von Diabetes eignen.

Die Erfindung betrifft daher Verbindungen der Formel I, 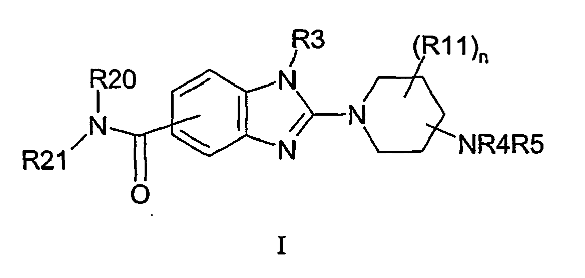 worin bedeuten
- R20: H, (C₁-C₃)-Alkyl, wobei (C₁-C₃)-Alkyl ein- oder mehrfach substituiert ist mit CN, NO₂, SH, OH, (C₁-C₆)-Alkyl, O-(C₁-C₆)-Alkyl oder S-(C₁-C₆)-Alkyl; (C₄-C₆)-Alkyl, wobei (C₄-C₆)-Alkyl ein- oder mehrfach substituiert ist mit F, Cl, Br, I, CN, NO₂, SH, OH, CF₃, (C₁-C₆)-Alkyl, O-(C₁-C₆)-Alkyl oder S-(C₁-C₆)- Alkyl; (C₇-C₁₀)-Alkyl, (C₃-C₁₀)-Cycloalkyl, (C₂-C₁₀)-Alkenyl, (C₂-C₁₀)-Alkinyl, (C₆- C₁₀)-Aryl, Heterocyclyl, (C₁-C₆)-Alkylen-(C₆-C₁₀)-Aryl, wobei (C₁-C₆)-Alkylen- (C₆-C₁₀)-Aryl (C₁-C₆)-Atkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl- Reste ein- oder mehrfach substituiert sein können mit F, Cl, Br, I, CN, NO₂, SH, OH, CF₃, (C₁-C₆)-Alkyl, O-(C₁-C₆)-Alkyl oder S-(C₁-C₆)-Alkyl;
- R21: (C₁-C₃)-Alkyl, wobei (C₁-C₃)-Alkyl ein- oder mehrfach substituiert ist mit CN, NO₂, SH, OH, (C₁-C₆)-Alkyl, O-(C₁-C₆)-Alkyl oder S-(C₁-C₆)-Alkyl; (C₄-C₆)-Alkyl, wobei (C₄-C₆)-Alkyl ein- oder mehrfach substituiert ist mit F, Cl, Br, I, CN, NO₂, SH, OH, CF₃, (C₁-C₆)-Alkyl, O-(C₁-C₆)-Alkyl oder S-(C₁-C₆)- Alkyl, (C₇-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, wobei (C₁-C₆)- Alkylen-(C₆-C₁₀)-Aryl (C₁-C₆)-Alkylen-(C₆-C₁₀)-Heteroeyelyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, CF₃, (C₁-C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
- R3: (C₁-C₆)-Alkyl, (C₃-C₇)-Cycloalkyl, (C₃-C₇)-Cycloalkyl-(C₁-C₃)-alkyl, Phenyl, Pyridyl, wobei die Alkyl, Cycloalkyl, Cycloalkyl-alkyl, Phenyl und Pyridylreste ein oder mehrfach mit CN, NO₂, -CF₃, -OCF₃, OR12, NR7R8, NR7CONR7R8, COR7, OCOR7, OCOOR7, COOR7, CONR7R8 oder OCONR7R8 substituiert sind; (C₂-C₁₀)-Alkenyl, (C₂-C₁₀)-Alkinyl, (C₆-C₁₀)-Aryl, Heterocyclyl, oder (C₁-C₆)- Alkylen-(C₆-C₁₀)-Aryl, wobei die Alkenyl-, Alkinyl-, Aryl, Heterocyclyl- und Alkylen-Aryl Reste ein- oder mehrfach substituiert sein können mit F, Cl, Br, I, CN, NO₂, OH, (C₁-C₆)-Alkyl, -CF₃, -OCF₃, OR7, NR7R8, NR7CONR7R8, COR7, OCOR7, OCOOR7, COOR7, CONR7R8 oder OCONR7R8;
wobei von dem Rest R3 die Bedeutungen unsubstituiertes Phenyl und unsubstituiertes Pyridyl sowie Phenyl und Pyridyl jeweils substituiert mit F, Cl, Br, I, OH, oder (C₁-C₆)-Alkyl ausgenommen sind;
- R7, R8: unabhängig voneinander H, (C₁-C₆)-Alkyl, -CF₃, (C₃-C₁₀)-Cycloalkyl, (C₆-C₁₀)- Aryl, Heterocyclyl, (C₁-C₆)-Alkylen-CONR9R10, CONR9R10, (C₁-C₆)-Alkylen- COOR9, COOR9, COR9, (C₁-C₆)-Alkylen-COR9, (C₁-C₆)-Alkylen-OR9, (C₁- C₆)-Alkylen-NR9R10, (C₁-C₆)-Alkylen-SR9, (C₁-C₆)-Alkylen-S(O)R9, (C₁-C₆)- Alkylen-S(O)₂R9, S(O)R9, S(O)₂R9, (C₁-C₄)-Akylen-(C₆-C₁₀)-Aryl oder (C₁- C₄)-Alkylen-Heterocyclyl;
- R9, R10: unabhängig voneinander H, (C₁-C₆)-Alkyl, (C₁-C₆)-Alkylen-(C₆-C₁₀)-Aryl, -(C₆- C₁₀)-Aryl, Heterocyclyl oder (C₁-C₆)-Alkylen-Heterocyclyl;
- R12: (C₁-C₆)-Alkyl, -CF₃, (C₃-C₁₀)-Cycloalkyl, (C₆-C₁₀)-Aryl, Heterocyclyl, (C₁-C₆)- Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁- C₆)-Alkylen-SR9, (C₁-C₆)-Alkylen-S(O)R9, (C₁-C₆)-Alkylen-S(O)₂R9, S(O)R9, S(O)₂R9, (C₂-C₄)-Alkylen-(C₆-C₁₀)-Aryl oder (C₁-C₄)-Alkylen-Heterocyclyl;
- R4, R5: unabhängig voneinander H, (C₁-C₆)-Alkyl oder (C₃-C₈)-Cykloalkyl, wobei (C₁- C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)2, OH, O(C₁-C₆)- Alkyl, OAryl, OHeteroaryl, S(C₁-C₆)-Alkyl, S(O)(C₁-C₆)-Alkyl oder S(O)₂(C₁- C₆)-Alkyl, wobei diese Alkylgruppen wiederum mit F, Cl, Br oder I substituiert sein können;
- R11: H, F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- n: 0, 1 oder 2;
sowie deren physiologisch verträglichen Salze.

Bevorzugt sind Verbindungen der Formel I, worin ein oder mehrere Reste die folgende Bedeutung haben:
- R20: H;
- R21: (C₃-C₁₀)-Cycloalkyl, (C₁-C₆)-Alkylen-(C₆-C₁₀)-Aryl, Phenyl, (C₁-C₆)-Alkylen- (C₆-C₁₀)-Pyridyl, wobei die Cycloalkyl-, Aryl- und Pyridyl-Reste ein- oder mehrfach substituiert sein können mit F, Cl, Br, I, CN, NO₂, SH, OH, CF₃, (C₁- C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
- R3: (C₁-C₆)-Alkyl, (C₃-C₇)-Cycloalkyl, (C₃-C₇)-Cycloalkyl-(C₁-C₃)-alkyl, Phenyl, Pyridyl, wobei die Alkyl, Cycloalkyl, Cycloalkyl-alkyl, Phenyl und Pyridylreste ein oder mehrfach mit CN, NO₂, -CF₃, -OCF₃, OR12, NR7R8, NR7CONR7R8, COR7, OCOR7, OCOOR7, COOR7, CONR7R8 oder OCONR7R8 substituiert sind; (C₂-C₁₀)-Alkenyl, (C₂-C₁₀)-Alkinyl, (C₆-C₁₀)-Aryl, Heterocyclyl, oder (C₁-C₆)- Alkylen-(C₆-C₁₀)-Aryl, wobei die Alkenyl-, Alkinyl-, Aryl, Heterocyclyl- und Alkylen-Aryl Reste ein- oder mehrfach substituiert sein können mit F, Cl, Br, I, CN, NO₂, OH, (C₁-C₆)-Alkyl, -CF₃, -OCF₃, OR7, NR7R8, NR7CONR7R8, COR7, OCOR7, OCOOR7, COLOR7, CONR7R8 oder OCONR7R8;
wobei von dem Rest R3 die Bedeutungen unsubstituiertes Phenyl und unsubstituiertes Pyridyl sowie Phenyl und Pyridyl jeweils substituiert mit F, Cl, Br, I, OH, oder (C₁-C₆)-Akyl ausgenommen sind;
- R7, R8: unabhängig voneinander H, (C₁-C₆)-Alkyl, -CF₃, (C₃-C₁₀)-Cycloakyl, (C₆-C₁₀)- Aryl, Heterocyclyl, (C₁-C₆)-Alkylen-CONR9R10, CONR9R10, (C₁-C₆)-Alkylen- COOR9, COOR9, COR9, (C₁-C₆)-Alkylen-COR9, (C₁-C₆)-Akylen-OR9, (C₁- C₆)-Alkylen-NR9R10, (C₁-C₆)-Alkylen-SR9, (C₁-C₆)-Alkylen-S(O)R9, (C₁-C₆)- Alkylen-S(O)₂R9, S(O)R9, S(O)₂R9, (C₁-C₄)-Alkylen-(C₆-C₁₀)-Aryl oder (C₁- C₄)-Alkylen-Heterocyclyl;
- R9, R10: unabhängig voneinander H, (C₁-C₆)-Alkyl, (C₁-C₆)-Alkylen-(C₆-C₁₀)-Aryl, -(C₆- C₁₀)-Aryl, Heterocyclyl oder (C₁-C₆)-Alkylen-Heterocyclyl;
- R12: (C₁-C₆)-Alkyl, -CF₃, (C₃-C₁₀)-Cycloalkyl, (C₆-C₁₀)-Aryl, Heterocyclyl, (C₁-C₆)- Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁- C₆)-Alkylen-SR9, (C₁-C₆)-Alkylen-S(O)R9, (C₁-C₆)-Alkylen-S(O)₂R9, S(O)R9, S(O)₂R9, (C₂-C₄)-Alkylen-(C₆-C₁₀)-Aryl oder (C₁-C₄)-Alkylen-Heterocyclyl;
- R4, R5: unabhängig voneinander H, (C₁-C₆)-Alkyl oder (C₃-C₈)-Cykloalkyl, wobei (C₁- C₆)-Alkyl oder (C₃-C₈-Cykloalkyl substitiuert sein können mit F, Cl, Br, I, CN, Aryl, Heterocyclyl, NH², NH(C₁-C₆)-Alkyl, N((C₁-C₆)-Alkyl)₂, OH, O(C₁-C₆)- Alkyl, OAryl, OHeteroaryl, S(C₁-C₆)-Alkyl, S(O)(C₁-C₆)-Alkyl oder S(O)₂(C₁- C₆)-Alkyl, wobei diese Alkylgruppen wiederum mit F, Cl, Br oder I substituiert sein können;
- R11: H, F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- n: 0, 1 oder 2;
sowie deren physiologisch verträglichen Salze.

Besonders bevorzugt sind Verbindungen der Formel I, worin ein oder mehrere Reste die folgende Bedeutung haben:
- R20: H;
- R21: (C₃-C₁₀)-Cycloalkyl, (C₁-C₆)-Alkylen-(C₆-C₁₀)-Aryl, Phenyl, (C₁-C₆)-Alkylen- (C₆-C₁₀)-Pyridyl, wobei die Cycloalkyl-, Aryl- und Pyridyl-Reste ein- oder mehrfach substituiert sein können mit F, Cl, Br, I, CN, NO₂, SH, OH, CF₃, (C₁- C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
- R3: unabhängig (C₂-C₁₀)-Alkenyl oder (C₁-C₆)-Alkylen-(C₆-C₁₀)-Aryl, wobei die Alkenyl- und (C₁-C₆)-Alkylen-(C₆-C₁₀)-Aryl Reste ein- oder mehrfach substituiert sein können mit F, Cl, Br;
- R4, R5: H;
- n: 0;
sowie deren physiologisch verträglichen Salze.

Ganz besonders bevorzugt sind Verbindungen der Formel I, worin ein oder mehrere Reste die folgende Bedeutung haben:
- R20: H;
- R21: (C₃-C₁₀)-Cycloalkyl, Benzyl, Pyridyl, Phenyl, wobei die Alkyl-, Cycloalkyl-, und Benzyl-Reste ein- oder mehrfach substituiert sein können mit F, Cl, Br, I, CN, NO₂ SH, OH, (C₁-C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
- R3: unabhängig (C₂-C₁₀)-Alkenyl oder (C₁-C₆)-Alkylen-(C₆-C₁₀)-Aryl, wobei die Alkenyl- und (C₁-C₆)-Alkylen-(C₆-C₁₀)-Aryl Reste ein- oder mehrfach substituiert sein können mit F, Cl, Br;
- R4. R5: H:
- n: 0;
sowie deren physiologisch verträglichen Salze.

Die Erfindung bezieht sich auf Verbindungen der Formel I, in Form ihrer Razemate, razemischen Mischungen und reinen Enantiomeren sowie auf ihre Diastereomeren und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin. Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
P0₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)n-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus. SO₂-N((CH₂)ₙ-Aryl)₂, SO₂-N((CH₂)ₙ-Heterocyclus)₂ wobei n = 0-6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-AAyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)2, N(Aryl)-CO-(C₁-C₆)-Alkyl, N(Heterocyclus)-CO-(C₁-C₆)-Alkyl, N(Aryl)-COO-(C₁-C₆)Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heteroeyclus)-CO-NH-Aryl, N(Aryl)-CO-N((C₁-C₆)-Akyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl, 2-Methyl-but-2-en-4-yl. Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₂H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Hetemcyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Butinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₅)-Alkyl oder NH₂ substituiert sein kann; C(=NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(Cᵢ-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Akyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁₋C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁₋C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Allcyl)₂, N(Aryl)-CO-N((C₁-C₆)-Alkyl)-Aryl, N(Heterocyclus)-CO-N((C₁-C₆)-Akyl)-Aryl, N(Aryl)-CO-N-(Aryl)₂, N(Heterocyclus)-CO-N-(Aryl)₂, Aryl, O-(CH₂)ₙ-Aryland O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(₌NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter Heterocyclyl, Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocyclus bzw. der heterocyclische Rest mit Benzolkernen kondensiert ist. Der Heterocyclus bzw. der heterocyclische Rest kann aromatisch, gesättigt aliphatisch oder teilweise ungesättigt aliphatisch sein.

Geeignete Heterocyclyl-Reste, bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazoiyl; Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterocyclen.

Die Heterocyclischen Ringe bzw. Heterocyclische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Allcyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Akyl)-CO-N((C₁-C₆)-Akyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Akyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heteroeyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 ng pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I können allein, aber oder auch in Kombination mit weiteren Wirkstoffen verabreicht werden. Als weitere Wirkstoffe für die Kombinationspräparate sind geeignet:
Alle Antidiabetika, die in der Roten Liste 2005, Kapitel 12 genannt sind. Sie können mit den erfindungsgemäßen Verbindungen der Formel I insbesondere zur synergistischen Wirkungsverbesserung kombiniert werden. Die Verabreichung der Wirkstofflcombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder Apidra^{®}, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulfonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken (PPAR = peroxisome proliferator activated receptor, PXR = pregnane X receptor, ATP = adenosine triphosphat).

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht (HMGCoA = 3-hydroxy-3-methylglutaryl Coenzyme A).

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, WO 2004/000804, WO 2004/000803, WO 2004/000805, EP 0114531, US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausfiihrungsform der Erfindung werden die Verbindungen der Formel I in Kombination mit PPAR alfa Agonist, wie z.B. GW 9578, GW 7647, verabreicht.

Bei einer Ausführungsform der Erfindung werden die Verbindungen der Formel I in Kombination mit einem gemischten PPAR alfa/gamma Agonisten, wie z.B. GW 1536, AVE 8042, AVE 8134, AVE 0847, oder wie in WO 00/64888, WO 00/64876, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht (MTP = Microsomal triglyceride transfer protein).

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht (CETP = cholesteryl ester transfer protein).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht (LDL = Low Density Lipids).

Bei einer Ausführungsform der Erfindung werden die Verbindungen der Formel I in Kombination mit einem ACAT-Inhibitor, wie z.B. Avasimibe, verabreicht (ACAT = acyl-Coenzyme A:cholesterol acyltransferase).

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulfonylharnstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxi]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.

Bei einer Ausfubrungsform werden die Verbindungen der Formel I in Kombination mit einem Adenosin A1 Agonisten, wie z. B. solchen, die in der WO 2004/003002 beschrieben sind, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylhamstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten (NPY = Neuropeptid Y, z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (MC4 = Melanocortin 4 receptor, z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (H3 = Histamin Rezeptor, z.B. 3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten (TNF = Tumor Necrosis Factor), CRF-Antagonisten (CRF = corticotropin releasing factor, z.B. [2-Methyl-9-(2,4,6-trimethylphenyl)-9H-1,3,9-triaza-fluoren-4-yl]-dipropyl-amin (WO 00/66585)), CRF BP-Antagonisten (CRF-BP = corticotropin releasing factor -binding protein, z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxi)-ethylamino]-ethanol Hydrochlorid (WO 01/8345)), CB1 (Cannabinoid Rezeptor 1) Rezeptor Antagonisten (zB. Rimonabant oder die in WO 02/28346 genannten Wirkstoffe, MSH (Melanocyt-stimulierendes Hormon)-Agonisten, CCK-A (CCK-A = cholecystokinin-A) Agonisten (z.B. {2-[4-(4-Chloro-2,5-dimethoxi-phenyl)-5-(2-cyclohexylethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten (Serotoninmimetika), z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxi-1-(2-diisopropylamino-ethylcarbamoyl)-3,4-dihydro-1H-isochinolin-2-carbonsäuretertiärbutylester (WO 01/85695)), TRH-Agonisten (TRH = TSH Releasing Hormone; TSH = thyroidstimulating hormone; thyrotropin ), siehe z.B. EP 0 462 884 entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (DA = Dopamine Autoreceptor, wie z.B. Bromocriptin, Doprexin), Lipase/Amylase-Inhibitoren (z.B. WO 00/40569), PPAR-Modulatoren (z.B. WO 00/78312), RXR- (RXR = Retinoid X Receptor) Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.); Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 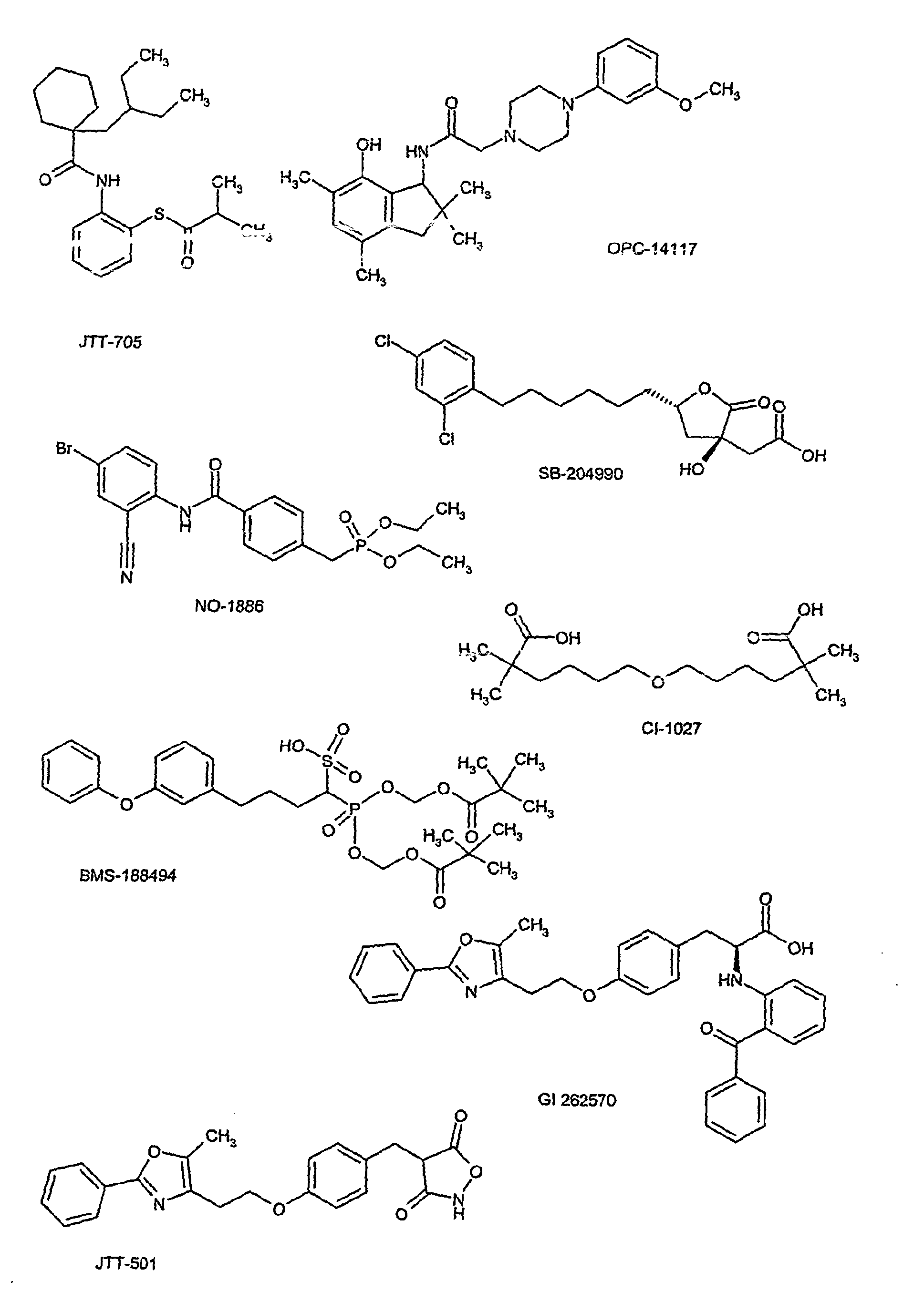 Die Verbindungen der Formel I lassen sich dadurch herstellen, dass man geeignete Ausgangsstoffe der Formel II, worin X eine Austrittsgruppe, wie Chlor, Brom, Jod, Sulfonyloxi, Sulfinyl oder Sulfoxyl, und R30 einen Ester oder ein Amid bedeutet, mit einer Verbindung der Formel IV ggf, in Gegenwart von geeigneten Basen und in geeigneten Lösemitteln umsetzt.

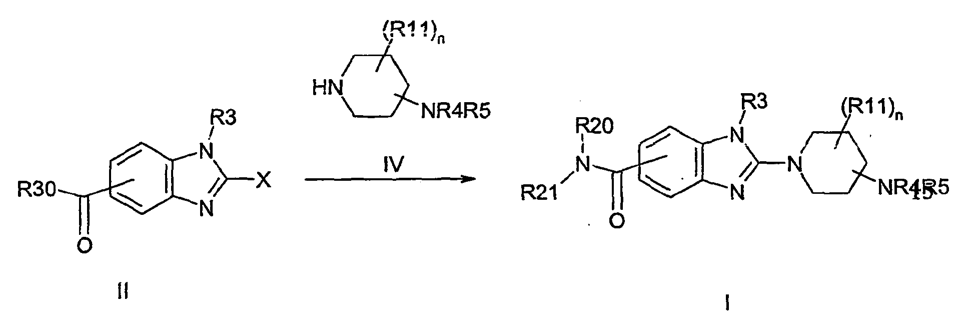

In den Fällen, wo R4 und R5 gleichzeitig Wasserstoff bedeutet, kann es zweckmäßig sein, den Rest IV in an der Stickstofffunktion geschützter Form einzusetzen und die Schutzgruppe nach erfolgter Reaktion mit IT wieder abzuspalten. Solche geeignete Schutzgruppen und die Verfahren der Einführung und Abspaltung sind bekannt (Siehe: Theodora W. Greene and Peter G. M. Wuts, Protective Groups in Organic Synthesis, 3rd Edition, John Wiley & Sons, Inc., New York, 1999)
Die Halogenverbindungen der Formel II können nach bekannten Verfahren wie beispielsweise durch Halogenierung der entsprechenden H-, Hydroxy- oder Thio-Verbindung (Formel II, X = H, OH oder SH) erhalten werden. Geeignete Halogenierungsmittel können beispielhaft Halogene, wie Chlor und Brom, N-Bromsuccinimid, Phosphorpentachlorid oder Phosphoroxychlorid sein.
Die Synthese von Verbindungen der Formel II ist in der Literatur beschrieben. Sie können beispielsweise durch Kondensation von substituierten Diaminobenzolderivaten mit Aldehyden in Gegenwart eines Oxidationsmitteln (z.B. Luftsauerstoff, Sauerstoff, Iod, Oxone, Chinonen, Peroxiden, usw.) oder alternativ mit Carbonsäuren, Nitrilen oder Amiden ohne oder in Gegenwart eines Katalysators hergestellt werden.
Die Synthese der Amine IV kann nach literaturbekannten Verfahren erfolgen.

Einige Derivate der Formel IV, wie zum Beispiel Piperidin-3-ylamine, sind kommerziell erhältlich.
Die Ester oder Amide CO-R30 können nach literaturbekannten Methoden in die Amide CO-NR20R21 überführt werden.

Die nachfolgend aufgeführten tabellarischen Beispiele dienen zur Erläuterung der Erfindung, ohne diese jedoch einzuschränken.

**Tabelle1:**

| | | | | | | | | |
|---|---|---|---|---|---|---|---|---|
| 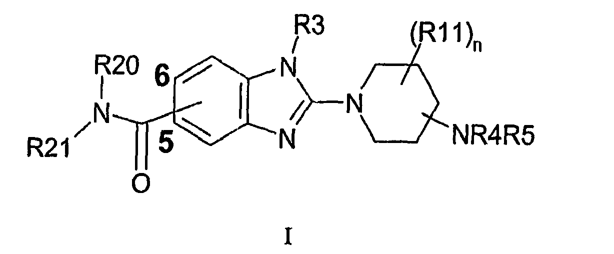 | | | | | | | | |

| Bsp. | A-Nr. | Bindungsstelle C=O-NR20R21 | R20 | R21 | R3 | NR4R5 | n | R11 |
|---|---|---|---|---|---|---|---|---|
| 1 | A003394933A | 6 | H | 4-Cl-Benzyl- | 4-F-Benzyl | 3-R-NH₂ x HCl | 0 | - |
| 2 | A003394934A | 6 | H | 3-CF₃-Benzyl- | 4-F-Benzyl | 3-R-NH₂ x HCl | 0 | - |
| 3 | A003394935A | 6 | H | 2-Cl-Benzyl- | 4-F-Benzyl | 3-R-NH₂ x HCl | 0 | - |
| 4 | A003394936A | 6 | H | Cyclohexyl- | 4-F-Benzyl | 3-R-NH₂ x HCl | 0 | - |
| 5 | A003394937A | 6 | H | 3-Pyridyl-CH₂- | 4-F-Benzyl | 3-R-NH₂ x HCl | 0 | - |
| 6 | A003395756A | 5 | H | 4-Cl-Benzyl- | 4-F-Benzyl | 3-R-NH₂ x HCl | 0 | - |
| 7 | A003395757A | 5 | H | 2-Cl-Benzyl- | 4-F-Benzyl | 3-R-NH₂ x HCl | 0 | - |
| 8 | A003395758A | 5 | H | 3-Pyridyl-(CH₂)₂- | 4-F-Benzyl | 3-R-NH₂ x HCl | 0 | - |
| 9 | A003395760A | 5 | H | 3-Cl-Benzyl- | 4-F-Benzyl | 3-R-NH₂ x HCl | 0 | - |
| 10 | A003401196A | 6 | H | Cyclopentyl- | -CH₂-CH=C(CH₃)₂ | 3-R-NH₂ x HCl | 0 | - |
| 11 | A003401273A | 6 | H | 4-F-Phenyl- | -CH₂-CH=C(CH₃)₂ | 3-R-NH₂ x HCl | 0 | - |
| 12 | A003401848A | 6 | H | 4-F-Phenyl- | 4-F-Benzyl | 3-R-NH₂ x HCl | 0 | - |

Die Verbindungen der Formel I zeichnen sich durch günstige Wirkungen auf den Lipid- und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV (Dipeptidyl Peptidase IV) Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen und zur Behandlung von Drogenmissbrauch.
Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:
Messung der DPP-IV Aktivität:
Material:
   DPP-IV aus Schweineniere (Sigma, München)
   H-Ala-Pro-AFC (Bachem, Weil am Rhein)
Testbedingungen:
   DPP-IV (1 mU/ml, Endkonzentration)
   H-Ala-Pro-AFC (15 µM Endkonzentration)
   in Tris/HCl (40 mM, pH 7.4), Gesamtvolumen 0,2 ml

Die Reaktion wurde bei Raumtemperatur für unterschiedliche Zeiträume (typischerweise 10 Minuten) durchgeführt und am Ende der Reaktion durch Zugabe von 20 µl ZnCl₂ (1 M) gestoppt. Der Umsatz von H-Ala-Pro-AFC wurde fluorimetrisch durch Messung der Emission bei 535 nm nach Anregung bei 405 nm bestimmt. Im Falle der Zugabe von Inhibitoren wurde das zugegebene Puffervolumen so angepasst, dass ein Gesamtvolumen der Testmischung von 200 µl eingehalten wurde.
IC₅₀ Werte für Inhibitoren wurden durch Variation der Inhibitorkonzentrationen bei der angegebenen Substratkonzentration von 15 µM bestimmt. Kᵢ und Kₘ Werte wurden durch enstsprechende Variation von Substrat- und Inhibitorkonzentration wie beschrieben (Dixon, M. and Webb, E.C.(1979) Enzymes, third edition, pp. 47-206, Academic Press) ermittelt. Die Werte für Kₘ, IC₅₀ and Kᵢ wurden mit einem kommerziell erhältlichen Software-Paket errechnet (Leatherbarrow, R.J. (1992) GraFit Version 3.0, Erithacus Software Ltd. Staines, U.K.).

**Tabelle 2: Biologische Aktivität der Beispiele:**

| Beispiel | IC₅₀ [µM] |
|---|---|
| 2 | 1,0 |
| 5 | 2,5 |
| 6 | 4,3 |
| 11 | 1,8 |

Aus der Tabelle ist abzulesen, dass die Verbindungen der Formel I die Aktivität der DPP-IV (Dipeptidyl Peptidase IV) hemmen und dadurch zur Senkung des Blutzuckerspiegels gut geeignet sind.

Nachfolgend wird die Herstellung einiger Ausführungsbeispiele detailliert beschrieben, die übrigen Verbindungen der Formel I wurden analog erhalten:

### Beispiel 1

### R-2-(3-Amino-piperidin-1-yl)-1-(4-fluoro-benzyl)-1H-benzimidazole-6-carbonsäure-4-chlorbenzylamid Hydrochlorid

### a) 2-Hydroxi-1H-benzimidazole-5-carbonsäure-methylester

Zur Lösung von 5 g 3,4-Diamino-benzoesäure-methylester in 10 ml N-Methylpyrrolidon wird die Lösung von 5,9 g Carbonyldiimidazol in 10 ml N-Methylpyrrolidon getropft und die Mischung 2 Stunden auf 100°C erwärmt. Nach dem Abkühlen auf 60°C wurden 5 ml Methanol zugetropft und anschließend die Mischung bei Raumtemperatur mit 150 ml Wasser verrührt, der Niederschlag abgesaugt, mit Isopropanol gewaschen und im Vakuum getrocknet.
Ausbeute: 4,0 g Fp.: 312°C

### b) 2-Chlor-1H-benzimidazol-5-carbonsäure-methylester

3,9 g 2-Hydroxi-1H-benzimidazol-5-carbonsäure-methylester und 50 ml Phosphoroxichlorid werden 6 Stunden bei 100°C gerührt. Der resultierende dicke Brei wird nach dem Erkalten vorsichtig in 500 ml Eiswasser eingerührt und der Feststoff nach einer Stunde abgesaugt, mit Wasser gewaschen und im Vakuum getrocknet.
Ausbeute: 4,3 g Fp.: 276°C

### c) Gemisch aus 2-Chlor-1-(4-fluor-benzyl)-1H-benzimidazol-6-carbonsäure-methylester und 2-Chlor-1-(4-fluor-benzyl)-1H-benzimidazol-5-carbonsäure-methylester

4,3 g 2-Chlor-1H-benzimidazol-6-carbonsäure-methylester werden in 15 ml N-Methylpyrrolidon gelöst, mit 4,2 g Pottasche versetzt und 60 Minuten bei Raumtemperatur gerührt. Dann werden 4,86 g 4-Fluorbenzylbromid zugefügt und die Mischung 4 Stunden bei 70°C gerührt, abgekühlt und mit 50 ml Wasser und 10 ml Eisessig verrührt. Der Feststoff wird abgesaugt und im Vakuum getrocknet.
Ausbeute: 4,0 g Fp. Harz

### d) Gemisch von R-2-(3-tert-Butoxycarbonylamino-piperidin-1-yl)-1-(4-fluor-benzyl)-1H-benzimidazol-6-carbonsäure-methylester und 2-(3-tert-Butoxycarbonylamino-piperidin-1-yl)-1-(4-fluor-benzyl)-1H-benzimidazol-5-carbonsäure-methylester

Das Gemisch aus 2-Chlor-1-(4-ffuor-benzyl)-1H-benzimidazol-6-carbonsäure-methylester und 2-Chlor-1-(4-fluoro-benzyl)-1H-benzimidazol-5-carbonsäure-methylester (insgesamt 2 g), 15 ml N-Methylpyrrolidon, 3 ml Triethylamin und 2,5 g R-Piperidin-3-yl-carbaminsäure-tert-butylester wird 6 Stunden auf 100°C erhitzt. Nach dem Erkaltenlassen wird die Mischung mit 50 ml Wasser versetzt und verrührt. Vom halbfesten Niederschlag wird abdekantiert, erneut in 5 ml Methanol aufgenommen und wieder mit 30 ml Wasser gefällt und verrührt. Der Feststoff wird abgesaugt und im Vakuum getrocknet.
Ausbeute: 2,1 g Fp.: Harz

### e) R-2-(3-tert-Butoxycarbonylamino-piperidin-1-yl)-1-(4-fluor-benzyl)-1H-benzimidazol-6-carbonsäure

Das Gemisch von insgesamt 2 g R-2-(3-tert-Butoxycarbonylamino-piperidin-1-yl)-1-(4-fluorbenzyl)-1H-benzimidazol-6-carbonsäure-methylester und R-2-(3-tert-Butoxycarbonylaminopiperidin-1-yl)-1-(4-ffuor-benzyl)-1H-benzimidazol-5-carbonsäure-methylester wird im Lösemittelgemisch von 10 ml Methanol, 20 ml Wasser und 10 ml Tetrahydrofuran mit 500 mg Lithiumhydroxid versetzt und 24 Stunden bei Raumtemperatur gerührt. Nach Zugabe von 2 ml Eisessig wurden die flüchtigen Anteile im Vakuum entfernt, der Rückstand mit Wasser verrührt und der Feststoff abgesaugt und anschließend säulenchromatografisch (Kieselgel, Laufmittel: Methylenchlorid: Isopropanol = 94:6) getrennt, wobei das 5-Isomere zuerst eluiert wird.
Ausbeute: 980 mg Fp.: Harz

### f) R-{1-[6-(4-Chloro-benzylcarbamoyl)-1-(4-fluoro-benzyl)-1H-benzimidazol-2-yl]-piperidin-3-yl}-carbaminsäure-tert-butylester (F-34529-074)

Die Lösung von 50 mg R-2-(3-tert-Butoxycarbonylamino-piperidin-1-yl)-1-(4-fluor-benzyl)-1H-benzimidazol-6-carbonsäure in 1,5 ml Dimethylformamid wurde nacheinander mit 40,6 mg HATU, 18 µl Triethylamin und 14 µl 4-Chlorbenzylamin versetzt und über Nacht bei Raumtemperatur gerührt. Die Mischung wurde dann mit 5 ml Wasser verdünnt und der Niederschlag abgesaugt und im Vakuum bei 40°C getrocknet.
Ausbeute: 30 mg Fp.: Harz

### g) R-2-(3-Amino-piperidin-1-yl)-1-(4-fluoro-benzyl)-1H-benzimidazole-6-carbonsäure-4-chlorbenzylamid Hydrochlorid (F-34529-083)

Die Lösung von 63 mg {1-[6-(4-Chlor-benzyl)-carbamoyl-1-(4-fluor-benzyl)-1H-benzimidazol-2-yl]-piperidin-3-yl}-carbaminsäure-tert-butylester in 1 ml Essigester wird mit 0,2 ml einer gesättigten HCl-Lösung in Essigsäureethylester versetzt und über Nacht bei Raumtemperatur stehen gelassen. Die flüchtigen Anteile wurden am Rotationsverdampfer abgezogen und der Rückstand in wenig Methylenchlorid gelöst und mit Diisopropylether ausgefällt. Der Feststoff wurde abgesaugt und im Vakuum bei 40°C getrocknet.
Ausbeute: 15 mg Fp.: 197,1°C

## Patentansprüche

1. Verbindungen der Formel I, 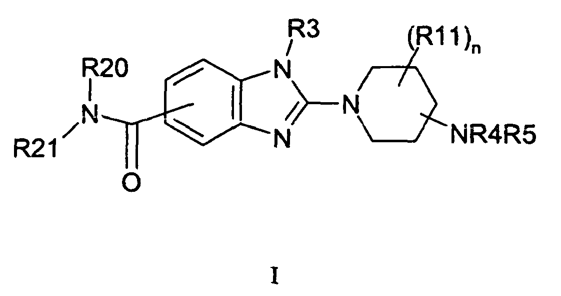 worin bedeuten
R20 H, (C₁-C₃)-Alkyl, wobei (C₁-C₃)-Alkyl ein- oder mehrfach substituiert ist mit CN, NO₂, SH, OH, (C₁-C₆)-Alkyl, O-(C₁-C₆)-Alkyl oder S-(C₁-C₆)-Alkyl; (C₄-C₆)-Alkyl, wobei (C₄-C₆)-Alkyl ein- oder mehrfach substituiert ist mit F, Cl, Br, I, CN, NO₂, SH, OH, CF₃, (C₁-C₆)-Alkyl, O-(C₁-C₆)-Alkyl oder S-(C₁-C₆)- Alkyl; (C₇-C₁₀)-Alkyl, (C₃-C₁₀)-Cycloalkyl, (C₂-C₁₀)-Alkenyl, (C₂-C₁₀)-Alkinyl, (C₆- C₁₀)-Aryl, Heterocyclyl, (C₁-C₆)-Alkylen-(C₆-C₁₀)-Aryl, wobei (C₁-C₆)-Alkylen- (C₆-C₁₀)-Aryl (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl- Reste ein- oder mehrfach substituiert sein können mit F, Cl, Br, I, CN, NO₂, SH, OH, CF₃, (C₁-C₆)-Alkyl, O-(C₁-C₆)-Alkyl oder S-(C₁-C₆)-Alkyl;
R21 (C₁-C₃)-Alkyl, wobei (C₁-C₃)-Alkyl ein- oder mehrfach substituiert ist mit CN, NO₂, SH, OH, (C₁-C₆)-Alkyl, O-(C₁-C₆)-Alkyl oder S-(C₁-C₆)-Alkyl; (C₄-C₆)-Alkyl, wobei (C₄-C₆)-Alkyl ein- oder mehrfach substituiert ist mit F, Cl, Br, I, CN, NO₂, SH, OH, CF₃, (C₁-C₆)-Alkyl, O-(C₁-C₆)-Alkyl oder S-(C₁-C₆)- Alkyl; (C₇-C₁₀)-Alkyl, (C₃-C₁₀)-Cycloalkyl, (C₂-C₁₀)-Alkenyl, (C₂-C₁₀)-Alkinyl, (C₆- C₁₀)-Aryl, Heterocyclyl, (C₁-C₆)-Alkylen-(C₆-C₁₀)-Aryl, wobei (C₁-C₆)-Alkylen- (C₆-C₁₀)-Aryl (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl- Reste ein- oder mehrfach substituiert sein können mit F, Cl, Br, I, CN, NO₂, SH, OH, CF₃, (C₁-C₆)-Alkyl, O-(C₁-C₆)-Alkyl oder S-(C₁-C₆)-Alkyl;
wobei von dem Rest R21 die Bedeutungen unsubstituiertes Phenyl una unsubstituiertes Benzyl ausgenommen sind;
R3 (C₁-C₆)-Allcyl, (C₃-C₇)-Cycloalkyl, (C₃-C₇)-Cycloalkyl-(C₁-C₃)-alkyl, Phenyl, Pyridyl, wobei die Alkyl, Cycloalkyl, Cycloalkyl-alkyl, Phenyl und Pyridylreste ein oder mehrfach mit CN, NO₂, -CF₃, -OCF₃, OR12, NR7R8, NR7CONR7R8, COR7, OCOR7, OCOOR7, COOR7, CONR7R8 oder OCONR7R8 substituiert sind; (C₂-C₁₀)-Alkenyl, (C₂-C₁₀)-Alkinyl, (C₆-C₁₀)-Aryl, Heterocyclyl, oder (C₁-C₆)- Alkylen-(C₆-C₁₀)-Aryl, wobei die Alkenyl-, Alkinyl-, Aryl, Heterocyclyl- und Alkylen-Aryl Reste ein- oder mehrfach substituiert sein können mit F, Cl, Br, I, CN, NO₂, OH, (C₁-C₆)-Alkyl, -CF₃, -OCF₃, OR7, NR7R8, NR7CONR7R8, COR7, OCOR7, OCOOR7, COOR7, CONR7R8 oder OCONR7R8;
wobei von dem Rest R3 die Bedeutungen unsubstituiertes Phenyl und unsubstituiertes Pyridyl sowie Phenyl und Pyridyl jeweils substituiert mit F, Cl, Br, I, OH, oder (C₁-C₆)-Alkyl ausgenommen sind;
R7, R8 unabhängig voneinander H, (C₁-C₆)-Alkyl, -CF₃, (C₃-C₁₀)-Cycloalkyl, (C₆-C₁₀)- Aryl, Heterocyclyl, (C₁-C₆)-Alkylen-CONR9R10, CONR9R10, (C₁-C₆)-Alkylen- COOR9, COOR9, COR9, (C₁-C₆)-Alkylen-COR9, (C₁-C₆)-Alkylen-OR9, (C₁- C₆)-Alkylen-NR9R10, (C₁-C₆)-Alkylen-SR9, (C₁-C₆)-Alkylen-S(O)R9, (C₁-C₆)- Alkylen-S(O)₂R9, S(O)R9, S(O)₂R9, (C₁-C₄)-Alkylen-(C₆-C₁₀)-Aryl oder (C₁- C₄)-Alkylen-Heterocyclyl;
R9, R10 unabhängig voneinander H, (C₁-C₆)-Alkyl, (C₁-C₆)-Alblen-(C₆-C₁₀)-Aryl, -(C₆- C₁₀)-Aryl, Heterocyclyl oder (C₁-C₆)-Alkylen-Heterocyclyl;
R12 (C₁-C₆)-Alkyl, -CF₃, (C₃-C₁₀)-Cycloalkyl, (C₆-C₁₀)-Aryl, Heterocyclyl, (C₁-C₆)- Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁- C₆)-Alkylen-SR9, (C₁-C₆)-Alkylen-S(O)R9, (C₁-C₆)-Alkylen-S(O)₂R9, S(O)R9, S(O)₂R9, (C₂-C₄)-Alkylen-(C₆-C₁₀)-Aryl oder (C₁-C₄)-Alkylen-Heterocyclyl,
R4, R5 unabhängig voneinander H, (C₁-C₆)-Alkyl oder (C₃-C₈)-Cykloalkyl, wobei (C₁- C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)₂, OH, O(C₁-C₆)- Alkyl, OAryl, OHeteroaryl, S(C₁-C₆)-Alkyl, S(O)(C₁-C₆)-Alkyl oder S(O)₂(C₁- C₆)-Alkyl, wobei diese Alkylgruppen wiederum mit F, Cl, Br oder I substituiert sein können;
R11 H, F, Cl, Br, I, (C₁-C₆₎-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
n 0, 1 oder 2;
sowie deren physiologisch verträglichen Salze.

2. Verbindungen der Formel I, gemäß Anspruch 1, **dadurch gekennzeichnet, dass** darin bedeuten
R20 H;
R21 (C₃-C₁₀)-Cycloalkyl, (C₁-C₆)-Alkylen-(C₆-C₁₀)-Aryl, Phenyl, (C₁-C₆)-Alkylen- (C₆-C₁₀)-Pyridyl, wobei die Cycloalkyl-, Aryl- und Pyridyl-Reste ein- oder mehrfach substituiert sein können mit F, Cl, Br, I, CN, NO₂, SH, OH, CF₃, (C₁- C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
R3 (C₁-C₆)-Alkyl, (C₃-C₇)-Cycloalkyl, (C₃-C₇)-Cycloalkyl-(C₁-C₃)-alkyl, Phenyl, Pyridyl, wobei die Alkyl, Cycloalkyl, Cycloalkyl-alkyl, Phenyl und Pyridylreste ein oder mehrfach mit CN, NO₂, -CF₃, -OCF₃, OR12, NR7R8, NR7CONR7R8, COR7, OCOR7, OCOOR7, COOR7, CONR7R8 oder OCONR7R8 substituiert sind; (C₂-C₁₀)-Alkenyl, (C₂-C₁₀)-Alkinyl, (C₆-C₁₀)-Aryl, Heterocyclyl, oder (C₁-C₆)- Alkylen-(C₆-C₁₀)-Aryl, wobei die Alkenyl-, Alkinyl-, Aryl, Heterocyclyl- und Alkylen-Aryl Reste ein- oder mehrfach substituiert sein können mit F, Cl, Br, I, CN, NO₂, OH, (C₁-C₆)-Alkyl, -CF₃, -OCF₃, OR7, NR7R8, NR7CONR7R8, COR7, OCOR7, OCOOR7, COOR7, CONR7R8 oder OCONR7R8;
wobei von dem Rest R3 die Bedeutungen unsubstituiertes Phenyl und unsubstituiertes Pyridyl sowie Phenyl und Pyridyl jeweils substituiert mit F, Cl, Br, I, OH, oder (C₁-C₆)-Alkyl ausgenommen sind;
R7, R8 unabhängig voneinander H, (C₁-C₆)-Alkyl, -CF₃, (C₃-C₁₀)-Cycloalkyl, (C₆-C₁₀)- Aryl, Heterocyclyl, (C₁-C₆)-Alkylen-CONR9R10, CONR9R10, (C₁-C₆)-Alkylen- COOR9, COOR9, COR9, (C₁-C₆)-Alkylen-COR9, (C₁-C₆)-Alkylen-OR9, (C₁- C₆)-Alkylen-NR9R10, (C₁-C₆)-Alkylen-SR9, (C₁-C₆)-Alkylen-S(O)R9, (C₁-C₆)- Alkylen-S(O)₂R9, S(O)R9, S(O)₂R9, (C₁-C₄)-Alkylen-(C₆-C₁₀)-Aryl oder (C₁- C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆- C₁₀)-Aryl, Heterocyclyl oder (C₁-C₆)-Alkylen-Heterocyclyl;
R12 (C₁-C₆)-Alkyl, -CF₃, (C₃-C₁₀)-Cycloalkyl, (C₆-C₁₀)-Aryl, Heterocyclyl, (C₁-C₆)- Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁- C₆)-Alkylen-SR9, (C₁-C₆)-Alkylen-S(O)R9, (C₁-C₆)-Alkylen-S(O)₂R9, S(O)R9, S(O)₂R9, (C₂-C₄)-Alkylen-(C₆-C₁₀)-Aryl oder (C₁-C₄)-Alkylen-Heterocyclyl;
R4, R5 unabhängig voneinander H, (C₁-C₆)-Alkyl oder (C₃-C₈)-Cykloalkyl, wobei (C₁- C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)₂, OH, O(C₁-C₆)- Alkyl, OAryl, OHeteroaryl, S(C₁-C₆)-Alkyl, S(O)(C₁-C₆)-Alkyl oder S(O)₂(C₁- C₆)-Alkyl, wobei diese Alkylgruppen wiederum mit F, Cl, Br oder I substituiert sein können;
R11 H, F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
n 0, 1 oder 2;
sowie deren physiologisch verträglichen Salze.

3. Verbindungen der Formel I, gemäß Anspruch 1 oder 2, **dadurch gekennzeichnet, dass** darin bedeuten
R20 H;
R21 (C₃-C₁₀)-Cycloalkyl, (C₁-C₆)-Alkylen-(C₆-C₁₀)-Aryl, Phenyl, (C₁-C₆)-Alkylen- (C₆-C₁₀)-Pyridyl, wobei die Cycloalkyl-, Aryl- und Pyridyl-Reste ein- oder mehrfach substituiert sein können mit F, Cl, Br, I, CN, NO₂, SH, OH, CF₃, (C₁- C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
R3 unabhängig (C₂-C₁₀)-Alkenyl oder (C₁-C₆)-Alkylen-(C₆-C₁₀)-Aryl, wobei die Alkenyl- und (C₁-C₆)-Alkylen-(C₆-C₁₀)-Aryl Reste ein- oder mehrfach substituiert sein können mit F, Cl, Br;
R4, R5 H;
n 0;
sowie deren physiologisch verträglichen Salze.

4. Verbindungen der Formel I, gemäß einem oder mehreren der Ansprüche 1 bis 3 , **dadurch gekennzeichnet, dass** darin bedeuten
R20 H;
R21 (C₃-C₁₀)-Cycloalkyl, Benzyl, Pyridyl, Phenyl, wobei die Alkyl-, Cycloalkyl-, und Benzyl-Reste ein- oder mehrfach substituiert sein können mit F, Cl, Br, I, CN, NO₂, SH, OH, (C₁-C₆)-Alkyl, O-(C₁-C₆)-Alkyl oder S-(C₁-C₆)-Alkyl;
wobei von dem Rest R21 die Bedeutungen unsubstituiertes Phenyl und unsubstituiertes Benzyl ausgenommen sind;
R3 unabhängig (C₂-C₁₀)-Alkenyl oder (C₁-C₆)-Alkylen-(C₆-C₁₀)-Aryl, wobei die Alkenyl- und (C₁-C₆)-Alkylen-(C₆-C₁₀)-Aryl Reste ein- oder mehrfach substituiert sein können mit F, Cl, Br;
R4, R5 H;
n 0;
sowie deren physiologisch verträglichen Salze.

5. Verbindungen gemäß einem oder mehreren der Ansprüche 1 bis 4 zur Anwendung als Arzneimittel.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA Reduktase Inhibitoren, Cholesterinresorptionsinhibitoren, PPAR gamma Agonisten, PPAR alfa Agonisten, PPAR alfa/gamma Agonisten, Fibrate, MTP-Inhibitoren, Gallensäureresorptionsinhibitoren, CFTP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 4 zur Herstellung eines Medikamentes zur Blutzuckersenkung.

10. Verwendung der Verbindungen gemäß einem oder mehreren der Ansprüche 1 bis 4 zur Herstellung eines Medikamentes zur Behandlung des Typ II Diabetes.

11. Verwendung der Verbindungen gemäß einem oder mehreren der Ansprüche 1 bis 4 zur Herstellung eines Medikamentes zur Behandlung von Lipid- und Kohlenhydratstoffwechselstörungen.

12. Verwendung der Verbindungen gemäß einem oder mehreren der Ansprüche 1 bis 4 zur Herstellung eines Medikaments zur Behandlung arteriosklerotischer Erscheinungen.

13. Verwendung der Verbindungen gemäß einem oder mehreren der Ansprüche 1 bis 4 zur Herstellung eines Medikaments zur Behandlung von Insulin Resistenz.

14. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 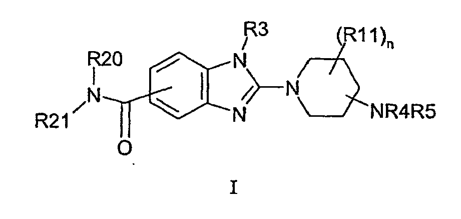 in which
R20 is H, (C₁-C₃)-alkyl, where (C₁-C₃)-alkyl is mono- or polysubstituted by CN, NO₂, SH, OH, (C₁-C₆)- alkyl, O-(C₁-C₆)-alkyl or S-(C₁-C₆)-alkyl; (C₄-C₆)-alkyl, where (C₄-C₆)-alkyl is mono- or polysubstituted by F, Cl, Br, I, CN, NO₂, SH, OH, CF₃, (C₁-C₆)-alkyl, O- (C₁-C₆) -alkyl or S- (C₁- C₆)-alkyl; (C₇-C₁₀)-alkyl, (C₃-C₁₀)-cycloalkyl, (C₂-C₁₀)- alkenyl, (C₂-C₁₀)-alkynyl, (C₆-C₁₀-aryl, heterocyclyl, (C₁-C₆)-alkylene-(C₆-C₁₀)-aryl, (C₁-C₆)-alkylene-(C₆-C₁₀)-heterocyclyl or S(O)₂- aryl, where the alkyl, cycloalkyl, alkenyl, alkynyl, alkylene, aryl and heterocyclyl radicals may be mono- or polysubstituted by F, Cl, Br, I, CN, NO₂, SH, OH, CF₃, (C₁-C₆)-alkyl, O-(C₁-C₆)-alkyl or S-(C₁-C₆)-alkyl;
R21 is (C₁-C₃)-alkyl, where (C₁-C₃)-alkyl is mono- or polysubstituted by CN, NO₂, SH, OH, (C₁-C₆)- alkyl, O-(C₁-C₆)-alkyl or S-(C₁-C₆)-alkyl; (C₄-C₆)-alkyl, where (C₄-C₆)-alkyl is mono- or polysubstituted by F, Cl, Br, I, CN, NO₂, SH, OH, CF₃, (C₁-C₆)-alkyl, O-(C₁-C₆)-alkyl or S-(C₁- C₆)-alkyl; (C₇-C₁₀)-alkyl, (C₃-C₁₀)-cycloalkyl, (C₂-C₁₀)- alkenyl, (C₂-C₁₀)-alkynyl, (C₆-C₁₀)-aryl, heterocyclyl, (C₁-C₆) -alkylene- (C₆-C₁₀) -aryl, (C₁-C₆)-alkylene-(C₆-C₁₀)-heterocyclyl or S(O)₂- aryl, where the alkyl, cycloalkyl, alkenyl, alkynyl, alkylene, aryl and heterocyclyl radicals may be mono- or polysubstituted by F, Cl, Br, I, CN, NO₂, SH, OH, CF₃, (C₁-C₆)-alkyl, O-(C₁-C₆)-alkyl or S-(C₁-C₆)-alkyl;
excluding the definitions of unsubstituted phenyl and unsubstituted benzyl from the R21 radical;
R3 is (C₁-C₆)-alkyl, (C₃-C₇)-cycloalkyl, (C₃-C₇)- cycloalkyl-(C₁-C₃)-alkyl, phenyl, pyridyl, where the alkyl, cycloalkyl, cycloalkylalkyl, phenyl and pyridyl radicals are mono- or polysubstituted by CN, NO₂, -CF₃, -OCF₃, OR12, NR7R8, NR7CONR7R8, COR7, OCOR7, OCOOR7, COOR7, CONR7R8 or OCONR7R8; (C₂-C₁₀)-alkenyl, (C₂-C₁₀)-alkynyl, (C₆-C₁₀)-aryl, heterocyclyl or (C₁-C₆)-alkylene-(C₆-C₁₀)-aryl, where the alkenyl, alkynyl, aryl, heterocyclyl and alkylenearyl radicals may be mono- or polysubstituted by F, Cl, Br, I, CN, NO₂, OH, (C₁-C₆)-alkyl, -CF₃, -OCF₃, OR7, NR7R8, NR7CONR7R8, COR7, OCOR7, OCOOR7, COOR7, CONR7R8 or OCONR7R8;
excluding the definitions of unsubstituted phenyl and unsubstituted pyridyl, and also phenyl and pyridyl each substituted by F, Cl, Br, I, OH or (C₁-C₆)-alkyl, from the R3 radical;
R7, R8 are each independently H, (C₁-C₆)-alkyl, -CF₃, (C₃-C₁₀)-cycloalkyl, (C₆-C₁₀)-aryl, heterocyclyl, (C₁-C₆)-alkylene-CONR9R1O, CONR9R10, (C₁-C₆)- alkylene-COOR9, COOR9, COR9, (C₁-C₆)-alkylene- COR9, (C₁-C₆)-alkylene-OR9, (C₁-C₆)-alkylene- NR9R10, (C₁-C₆)-alkylene-SR9, (C₁-C₆)-alkylene- S(O)R9, (C₁-C₆)-alkylene-S(O)₂R9, S(O)R9, S(O)₂R9, (C₁-C₄)-alkylene-(C₆-C₁₀)-aryl or (C₁- C₄)-alkylene-heterocyclyl;
R9, R10 are each independently H, (C₁-C₆)-alkyl, (C₁-C₆)- alkylene-(C₆-C₁₀)-aryl, -(C₆-C₁₀)-aryl, heterocyclyl or (C₁-C₆)-alkylene- heterocyclyl;
R12 is (C₁-C₆)-alkyl, -CF₃, (C₃-C₁₀)-cycloalkyl, (C₆- C₁₀)-aryl, heterocyclyl, (C₁-C₆)-alkylene- CONR9R10, CONR9R10, (C₁-C₆)-alkylene-COOR9, COOR9, COR9, (C₁-C₆)-alkylene-COR9, (C₁-C₆)- alkylene-OR9, (C₁-C₆)-alkylene-NR9R10, (C₁-C₆)- alkylene-SR9, (C₁-C₆)-alkylene-S(O)R9, (C₁-C₆)- alkylene-S(O)₂R9, S(O)R9, S(O)₂R9, (C₂-C₄)- alkylene-(C₆-C₁₀)-aryl or (C₁-C₄)-alkylene- heterocyclyl;
R4, R5 are each independently H, (C₁-C₆)-alkyl or (C₃- C₈)-cycloalkyl, where (C₁-C₆)-alkyl or (C₃-C₈)- cycloalkyl may be substituted by F, Cl, Br, I, CN, aryl, heterocyclyl, NH₂, NH(C₁-C₆)-alkyl, N((C₁-C₆)-alkyl)₂, OH, O(C₁-C₆)-alkyl, Oaryl, Oheteroaryl, S(C₁-C₆)-alkyl, S(O)(C₁-C₆)-alkyl or S(O)₂(C₁-C₆)-alkyl, where these alkyl groups may in turn be substituted by F, Cl, Br or I;
R11 is H, F, Cl, Br, I, (C₁-C₆)-alkyl, (C₃-C₈)- cycloalkyl, NH₂, NH(C₁-C₆)-alkyl, NH(C₃-C₇)- cycloalkyl, N((C₁-C₆)-alkyl)₂ or O-(C₁-C₆)-alkyl, where the alkyl groups may be mono- or polysubstituted by F, Cl, Br or I;
n is 0, 1 or 2;
and physiologically compatible salts thereof.

2. A compound of the formula I as claimed in claim 1, wherein
R20 is H;
R21 is (C₃-C₁₀)-cycloalkyl, (C₁-C₆)-alkylene-(C₆-C₁₀)- aryl, phenyl, (C₁-C₆)-alkylene-(C₆-C₁₀)-pyridyl, where the cycloalkyl, aryl and pyridyl radicals may be mono- or polysubstituted by F, Cl, Br, I, CN, NO₂, SH, OH, CF₃, (C₁-C₆)-alkyl, O- (C₁-C₆)-alkyl or S-(C₁-C₆)-alkyl;
excluding the definitions of unsubstituted phenyl and unsubstituted benzyl from the R21 radical;
R3 is (C₁-C₆)-alkyl, (C₃-C₇)-cycloalkyl, (C₃-C₇)- cycloalkyl-(C₁-C₃)-alkyl, phenyl, pyridyl, where the alkyl, cycloalkyl, cycloalkylalkyl, phenyl and pyridyl radicals are mono- or polysubstituted by CN, NO₂, -CF₃, -OCF₃, OR12, NR7R8, NR7CONR7R8, COR7, OCOR7, OCOOR7, COOR7, CONR7R8 or OCONR7R8; (C₂-C₁₀)-alkenyl, (C₂-C₁₀)-alkynyl, (C₆-C₁₀)-aryl, heterocyclyl or (C₁-C₆)-alkylene-(C₆-C₁₀)-aryl, where the alkenyl, alkynyl, aryl, heterocyclyl and alkylenearyl radicals may be mono- or polysubstituted by F, Cl, Br, I, CN, N0₂, OH, (C₁-C₆)-alkyl, -CF₃, -OCF₃, OR7, NR7R8, NR7CONR7R8, COR7, OCOR7, OCOOR7, COOR7, CONR7R8 or OCONR7R8;
excluding the definitions of unsubstituted phenyl and unsubstituted pyridyl, and also phenyl and pyridyl each substituted by F, Cl, Br, I, OH or (C₁-C₆)-alkyl, from the R3 radical;
R7, R8 are each independently H, (C₁-C₆)-alkyl, -CF₃, (C₃-C₁₀)-cycloalkyl, (C₆-C₁₀)-aryl, heterocyclyl, (C₁-C₆)-alkylene-CONR9RlO, CONR9R10, (C₁-C₆)- alkylene-COOR9, COOR9, COR9, (C₁-C₆)-alkylene- COR9, (C₁-C₆)-alkylene-OR9, (C₁-C₆)-alkylene- NR9R10, (C₁-C₆)-alkylene-SR9, (C₁-C₆)-alkylene- S(O)R9, (C₁-C₆)-alkylene-S(O)₂R9, S(O)R9, S(O)₂R9, (C₁-C₄)-alkylene-(C₆-C₁₀)-aryl or (C₁-C₄)- alkylene-heterocyclyl;
R9, R10 are each independently H, (C₁-C₆)-alkyl, (C₁-C₆)- alkylene-(C₆-C₁₀)-aryl, -(C₆-C₁₀)-aryl, heterocyclyl or (C₁-C₆)-alkylene- heterocyclyl;
R12 is (C₁-C₆)-alkyl, -CF₃, (C₃-C₁₀)-cycloalkyl, (C₆- C₁₀)-aryl, heterocyclyl, (C₁-C₆)-alkylene- CONR9R10, CONR9R10, (C₁-C₆)-alkylene-COOR9, COOR9, COR9, (C₁-C₆)-alkylene-COR9, (C₁-C₆)- alkylene-OR9, (C₁-C₆)-alkylene-NR9R10, (C₁-C₆)- alkylene-SR9, (C₁-C₆)-alkylene-S(O)R9, (C₁-C₆)- alkylene-S(O)₂R9, S(O)R9, S(O)₂R9, (C₂-C₄)- alkylene-(C₆-C₁₀)-aryl or (C₁-C₄)-alkylene- heterocyclyl;
R4, R5 are each independently H, (C₁-C₆)-alkyl or (C₃- C₈)-cycloalkyl, where (C₁-C₆)-alkyl or (C₃-C₈)- cycloalkyl may be substituted by F, Cl, Br, I, CN, aryl, heterocyclyl, NH₂, NH(C₁-C₆)-alkyl, N((C₁-C₆)-alkyl)₂, OH, O(C₁-C₆)-alkyl, Oaryl, Oheteroaryl, S(C₁-C₆)-alkyl, S(O)(C₁-C₆)-alkyl or S(O)₂(C₁-C₆)-alkyl, where these alkyl groups may in turn be substituted by F, Cl, Br or I;
R11 is H, F, Cl, Br, I, (C₁-C₆)-alkyl, (C₃-C₈)- cycloalkyl, NH₂, NH(C₁-C₆)-alkyl, NH(C₃-C₇)- cycloalkyl, N((C₁-C₆)-alkyl)₂ or O-(C₁-C₆)-alkyl, where the alkyl groups may be mono- or polysubstituted by F, Cl, Br or I;
n is 0, 1 or 2;
and physiologically compatible salts thereof.

3. A compound of the formula I as claimed in claim 1 or 2, wherein
R20 is H;
R21 is (C₃-C₁₀)-cycloalkyl, (C₁-C₆)-alkylene-(C₆-C₁₀)- aryl, phenyl, (C₁-C₆)-alkylene-(C₆-C₁₀)-pyridyl, where the cycloalkyl, aryl and pyridyl radicals may be mono- or polysubstituted by F, Cl, Br, I, CN, NO₂, SH, OH, CF₃, (C₁-C₆)-alkyl, O- (C₁-C₆)-alkyl or S-(C₁-C₆)-alkyl;
excluding the definitions of unsubstituted phenyl and unsubstituted benzyl from the R21 radical;
R3 is independently (C₂-C₁₀)-alkenyl or (C₁-C₆)- alkylene-(C₆-C₁₀)-aryl, where the alkenyl and (C₁-C₆)-alkylene-(C₆-C₁₀)-aryl radicals may be mono- or polysubstituted by F, Cl, Br;
R4, R5 are each H;
n is 0;
and physiologically compatible salts thereof.

4. A compound of the formula I as claimed in one or more of claims 1 to 3, wherein
R20 is H;
R21 is (C₃-C₁₀)-cycloalkyl, benzyl, pyridyl, phenyl, where the alkyl, cycloalkyl and benzyl radicals may be mono- or polysubstituted by F, Cl, Br, I, CN, NO₂, SH, OH, (C₁-C₆)-alkyl, O-(C₁-C₆)- alkyl or S-(C₁-C₆)-alkyl;
excluding the definitions of unsubstituted phenyl and unsubstituted benzyl from the R21 radical;
R3 is independently (C₂-C₁₀)-alkenyl or (C₁-C₆)- alkylene-(C₆-C₁₀)-aryl, where the alkenyl and (C₁-C₆)-alkylene-(C₆-C₁₀)-aryl radicals may be mono- or polysubstituted by F, Cl, Br;
R4, R5 are each H;
n is 0;
and physiologically compatible salts thereof.

5. A compound as claimed in one or more of claims 1 to 4 for use as a medicament.

6. A medicament comprising one or more of the compounds as claimed in one or more of claims 1 to 4.

7. A medicament comprising one or more of the compounds as claimed in one or more of claims 1 to 4 and at least one further active ingredient.

8. A medicament as claimed in claim 7, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin antagonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotonin and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptin, doprexin), lipase/amylase inhibitors, PPAR modulators, RXR modulators or TR-β agonists or amphetamines.

9. The use of the compounds as claimed in one or more of claims 1 to 4 for producing a medicament for lowering blood sugar.

10. The use of the compounds as claimed in one or more of claims 1 to 4 for producing a medicament for treating type II diabetes.

11. The use of the compounds as claimed in one or more of claims 1 to 4 for producing a medicament for treating lipid and carbohydrate metabolism disorders.

12. The use of the compounds as claimed in one or more of claims 1 to 4 for producing a medicament for treating arteriosclerotic manifestations.

13. The use of the compounds as claimed in one or more of claims 1 to 4 for producing a medicament for treating insulin resistance.

14. A process for producing a medicament comprising one or more of the compounds as claimed in one or more of claims 1 to 4, which comprises mixing the active ingredient with a pharmaceutically suitable carrier and bringing this mixture into a form suitable for administration.

## Revendications

1. Composés de formule I, 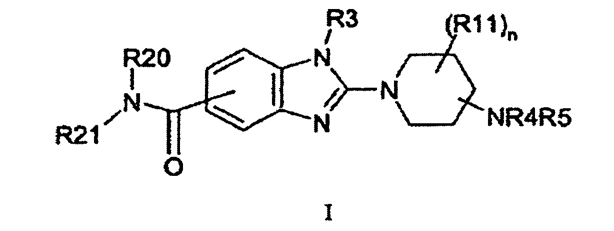 dans laquelle
R20 signifie H, (C₁-C₃)-alkyle, où (C₁-C₃)-alkyle est monosubstitué ou polysubstitué par CN, NO₂, SH, OH, (C₁-C₆)-alkyle, O-(C₁-C₆)-alkyle ou S-(C₁-C₆)- alkyle ; (C₄-C₆)-alkyle, où (C₄-C₆)-alkyle est monosubstitué ou polysubstitué par F, Cl, Br, I, CN, NO₂, SH, OH, CF₃, (C₁-C₆)-alkyle, O-(C₁-C₆)- alkyle ou S-(C₁-C₆)-alkyle ; (C₇-C₁₀)-alkyle, (C₃-C₁₀)-cycloalkyle, (C₂-C₁₀)- alcényle, (C₂-C₁₀)-alcynyle, (C₆-C₁₀)-aryle, (C₆- C₁₀)-hétérocyclyle, (C₁-C₆)-alkylène-(C₆-C₁₀)-aryle, (C₁-C₆)-alkylène-(C₆-C₁₀)-hétérocyclyle ou S(O)₂- aryle, où les radicaux alkyle, cycloalkyle, alcényle, alcynyle, alkylène, aryle et hétérocyclyle peuvent être monosubstitués ou polysubstitués par F, Cl, Br, I, CN, NO₂, SH, OH, CF₃, (C₁-C₆)-alkyle, O-(C₁-C₆)-alkyle ou S-(C₁-C₆)- alkyle ;
R21 signifie (C₁-C₃)-alkyle, où (C₁-C₃)-alkyle est monosubstitué ou polysubstitué par CN, NO₂, SH, OH, (C₁-C₆)-alkyle, O-(C₁-C₆)-alkyle ou S-(C₁-C₆)- alkyle ; (C₄-C₆)-alkyle, où (C₄-C₆)-alkyle est monosubstitué ou polysubstitué par F, Cl, Br, I, CN, NO₂, SH, OH, CF₃, (C₁-C₆)-alkyle, O-(C₁-C₆)- alkyle ou S-(C₁-C₆)-alkyle ; (C₇-C₁₀)-alkyle, (C₃-C₁₀)-cycloalkyle, (C₂-C₁₀)- alcényle, (C₂-C₁₀)-alcynyle, (C₆-C₁₀)-aryle, (C₆- C₁₀)-hétérocyclyle, (C₁-C₆)-alkylène-(C₆-C₁₀)-aryle, (C₁-C₆)-alkylène-(C₆-C₁₀)-hétérocyclyle ou S(O)₂- aryle, où les radicaux alkyle, cycloalkyle, alcényle, alcynyle, alkylène, aryle et hétérocyclyle peuvent être monosubstitués ou polysubstitués par F, Cl, Br, I, CN, NO₂, SH, OH, CF₃, (C₁-C₆)-alkyle, O-(C₁-C₆)-alkyle ou S-(C₁-C₆)- alkyle ;
où, pour le radical R21, les significations phényle non substitué et benzyle non substitué, sont exclues ;
R3 signifie (C₁-C₆)-alkyle, (C₃-C₇)-cycloalkyle, (C₃- C₇)-cycloalkyl-(C₁-C₃)-alkyle, phényle, pyridyle, où les radicaux alkyle, cycloalkyle, phényle et pyridyle sont monosubstitués ou polysubstitués par CN, NO₂, -CF₃, -OCF₃, OR12, NR7R8, NR7CONR7R8, COR7, OCOR7, OCOOR7, COOR7, CONR7R8 ou OCONR7R8 ; (C₂-C₁₀)-alcényle, (C₂-C₁₀)-alcynyle, (C₆-C₁₀)-aryle, (C₆-C₁₀)-hétérocyclyle, ou (C₁-C₆)-alkylène-(C₆-C₁₀)- aryle, où les radicaux alcényle, alcynyle, aryle, hétérocyclyle et alkylène-aryle peuvent être monosubstitués ou polysubstitués par F, Cl, Br, I, CN, NO₂, OH, (C₁-C₆)-alkyle, -CF₃, -OCF₃, OR7, NR7R8, NR7CONR7R8, COR7, OCOR7, OCOOR7, COOR7, CONR7R8 ou OCONR7R8 ;
où, pour le radical R3, les significations phényle non substitué et pyridyle non substitué ainsi que phényle et pyridyle, à chaque fois substitué par F, Cl, Br, I, OH, ou (C₁-C₆)-alkyle, sont exclues ;
R7, R8 signifient indépendamment l'un de l'autre, H, (C₁-C₆)-alkyle, -CF₃, (C₃-C₁₀)-cycloalkyle, (C₆-C₁₀)- aryle, (C₆-C₁₀)-hétérocyclyle, (C₁-C₆)-alkylène- CONR9R10, CONR9R10, (C₁-C₆)-alkylène-COOR9, COOR9, COR9, (C₁-C₆)-alkylène-COR9, (C₁-C₆)-alkylène-OR9, (C₁-C₆)-alkylène-NR9R10, (C₁-C₆)-alkylène-SR9, (C₁- C₆)-alkylène-S(O)R9, (C₁-C₆)-alkylène-S(O)₂R9, S(O)R9, S(O)₂R9, (C₁-C₄)-alkylène-(C₆-C₁₀)-aryle ou (C₁-C₄)-alkylène-hétérocyclyle ;
R9, R10 signifient, indépendamment l'un de l'autre H, (C₁-C₆)-alkyle, (C₁-C₆)-alkylène-(C₆-C₁₀)-aryle, -(C₆-C₁₀)-aryle, hétérocyclyle ou (C₁-C₆)-alkylène- hétérocyclyle ;
R12 signifie (C₁-C₆)-alkyle, -CF₃, (C₃-C₁₀)-cycloalkyle, (C₆-C₁₀)-aryle, hétérocyclyle, (C₁-C₆)-alkylène- CONR9R10, CONR9R10, (C₁-C₆)-alkylène-COOR9, COOR9, COR9, (C₁-C₆)-alkylène-COR9, (C₁-C₆)-alkylène-OR9, (C₁-C₆)-alkylène-NR9R10, (C₁-C₆)-alkylène-SR9, (C₁- C₆)-alkylène-S(O)R9, (C₁-C₆)-alkylène-S(O)₂R9, S(O)R9, S(O)₂R9, (C₂-C₄)-alkylène-(C₆-C₁₀)-aryle ou (C₁-C₄)-alkylène-hétérocyclyle ;
R4, R5 signifient, indépendamment l'un de l'autre H, (C₁-C₆)-alkyle ou (C₃-C₈)-cycloalkyle, où (C₁-C₆)- alkyle ou (C₃-C₈)-cycloalkyle peuvent être substitués par F, Cl, Br, I, CN, aryle, hétérocyclyle, NH₂, NH(C₁-C₆)-alkyle, N((C₁-C₆)- alkyle)₂, OH, O(C₁-C₆)-alkyle, O-aryle, O- hétéroaryle, S(C₁-C₆)-alkyle, S(O) (C₁-C₆)-alkyle ou S(O)₂(C₁-C₆)-alkyle, ces groupes alkyle pouvant à nouveau être substitués par F, Cl, Br ou I ;
R11 signifie H, F, Cl, Br, I, (C₁-C₆)-alkyle, (C₃-C₈)- cycloalkyle, NH₂, NH(C₁-C₆)-alkyle, NH(C₃-C₇)- cycloalkyle, N((C₁-C₆)-alkyle)₂ ou O-(C₁-C₆)-alkyle, où les groupes alkyle peuvent être monosubstitués ou polysubstitués par F, Cl, Br ou I ;
n vaut 0, 1 ou 2 ;
ainsi que leurs sels physiologiquement acceptables.

2. Composés de formule I, selon la revendication 1, **caractérisés en ce que**
R20 signifie H ;
R21 signifie (C₃-C₁₀)-cycloalkyle, (C₁-C₆)-alkylène-(C₆- C₁₀)-aryle, phényle, (C₁-C₆)-alkylène-(C₆-C₁₀)- pyridyle, où les radicaux cycloalkyle, aryle et pyridyle peuvent être monosubstitués ou polysubstitués par F, Cl, Br, I, CN, NO₂, SH, OH, CF₃, (C₁-C₆)-alkyle, O-(C₁-C₆)-alkyle ou S-(C₁-C₆)- alkyle ;
où, pour le radical R21, les significations phényle non substitué et benzyle non substitué, sont exclues ;
R3 signifie (C₁-C₆)-alkyle, (C₃-C₇)-cycloalkyle, (C₃- C₇)-cycloalkyl-(C₁-C₃)-alkyle, phényle, pyridyle, où les radicaux alkyle, cycloalkyle, cycloalkylalkyle, phényle et pyridyle sont monosubstitués ou polysubstitués par CN, NO₂, -CF₃, -OCF₃, OR12, NR7R8, NR7CONR7R8, COR7, OCOR7, OCOOR7, COOR7, CONR7R8 ou OCONR7R8 ; (C₂-C₁₀)-alcényle, (C₂-C₁₀)-alcynyle, (C₆-C₁₀)-aryle, hétérocyclyle, ou (C₁-C₆)-alkylène-(C₆-C₁₀)-aryle, où les radicaux alcényle, alcynyle, aryle, hétérocyclyle et alkylène-aryle peuvent être monosubstitués ou polysubstitués par F, Cl, Br, I, CN, NO₂, OH, (C₁-C₆)-alkyle, -CF₃, -OCF₃, OR7, NR7R8, NR7CONR7R8, COR7, OCOR7, OCOOR7, COOR7, CONR7R8 ou OCONR7R8 ;
où, pour le radical R3, les significations phényle non substitué et pyridyle non substitué ainsi que phényle et pyridyle, à chaque fois substitué par F, Cl, Br, I, OH, ou (C₁-C₆)-alkyle, sont exclues ;
R7, R8 signifient indépendamment l'un de l'autre, H, (C₁-C₆)-alkyle, -CF₃, (C₃-C₁₀)-cycloalkyle, (C₆-C₁₀)- aryle, hétérocyclyle, (C₁-C₆)-alkylène-CONR9R10, CONR9R10, (C₁-C₆)-alkylène-COOR9, COOR9, COR9, (C₁- C₆)-alkylène-COR9, (C₁-C₆)-alkylène-OR9, (C₁-C₆)- alkylène-NR9R10, (C₁-C₆)-alkylène-SR9, (C₁-C₆)- alkylène-S(O)R9, (C₁-C₆)-alkylène-S(O)₂R9, S(O)R9, S(O)₂R9, (C₁-C₄)-alkylène-(C₆-C₁₀)-aryle ou (C₁-C₄)- alkylène-hétérocyclyle ;
R9, R10 signifient, indépendamment l'un de l'autre H, (C₁-C₆)-alkyle, (C₁-C₆)-alkylène-(C₆-C₁₀)-aryle, -(C₆-C₁₀)-aryle, hétérocyclyle ou (C₁-C₆)-alkylène- hétérocyclyle ;
R12 signifie (C₁-C₆)-alkyle, -CF₃, (C₃-C₁₀)-cycloalkyle, (C₆-C₁₀)-aryle, hétérocyclyle, (C₁-C₆)-alkylène- CONR9R10, CONR9R10, (C₁-C₆)-alkylène-COOR9, COOR9, COR9, (C₁-C₆)-alkylène-COR9, (C₁-C₆)-alkylène-OR9, (C₁-C₆)-alkylène-NR9R10, (C₁-C₆)-alkylène-SR9, (C₁- C₆)-alkylène-S(O)R9, (C₁-C₆)-alkylène-S(O)₂R9, S(O)R9, S(O)₂R9, (C₂-C₄)-alkylène-(C₆-C₁₀)-aryle ou (C₁-C₄)-alkylène-hétérocyclyle ;
R4, R5 signifient, indépendamment l'un de l'autre H, (C₁-C₆)-alkyle ou (C₃-C₈)-cycloalkyle, où (C₁-C₆)- alkyle ou (C₃-C₈)-cycloalkyle peuvent être substitués par F, Cl, Br, I, CN, aryle, hétérocyclyle, NH₂, NH(C₁-C₆)-alkyle, N((C₁-C₆)- alkyle)₂, OH, O(C₁-C₆)-alkyle, O-aryle, O- hétéroaryle, S(C₁-C₆)-alkyle, S(O)(C₁-C₆)-alkyle ou S(O)₂(C₁-C₆)-alkyle, ces groupes alkyle pouvant à nouveau être substitués par F, Cl, Br ou I ;
R 11 signifie H, F, Cl, Br, I, (C₁-C₆)-alkyle, (C₃-C₈)- cycloalkyle, NH₂, NH(C₁-C₆)-alkyle, NH(C₃-C₇)- cycloalkyle, N((C₁-C₆)-alkyle)₂ ou O-(C₁-C₆)-alkyle, où les groupes alkyle peuvent être monosubstitués ou polysubstitués par F, Cl, Br ou I ;
n 0, 1 ou 2 ;
ainsi que leurs sels physiologiquement acceptables.

3. Composés de formule I, selon la revendication 1 ou 2, **caractérisés en ce que**
R20 signifie H ;
R21 signifie (C₃-C₁₀)-cycloalkyle, (C₁-C₆)-alkylène-(C₆- C₁₀-aryle, phényle, (C₁-C₆)-alkylène-(C₆-C₁₀)- pyridyle, où les radicaux cycloalkyle, aryle et pyridyle peuvent être monosubstitués ou polysubstitués par F, Cl, Br, I, CN, NO₂, SH, OH, CF₃, (C₁-C₆)-alkyle, O-(C₁-C₆)-alkyle ou S-(C₁-C₆)- alkyle ;
où, pour le radical R21, les significations phényle non substitué et benzyle non substitué, sont exclues ;
R3 signifie, indépendamment, (C₂-C₁₀)-alcényle ou (C₁- C₆)-alkylène-(C₆-C₁₀)-aryle, où les radicaux alcényle et (C₁-C₆)-alkylène-(C₆-C10)-aryle peuvent être monosubstitués ou polysubstitués par F, Cl, Br ;
R4, R5 signifient H ;
n vaut 0 ;
ainsi que leurs sels physiologiquement acceptables.

4. Composés de formule I selon l'une ou plusieurs des revendications 1 à 3, **caractérisés en ce que**
R20 signifie H ;
R21 signifie (C₃-C₁₀)-cycloalkyle, benzyle, pyridyle, phényle, où les radicaux alkyle, cycloalkyle, et benzyle peuvent être monosubstitués ou polysubstitués par F, Cl, Br, I, CN, NO₂, SH, OH, (C₁-C₆)-alkyle, O-(C₁-C₆)-alkyle ou S-(C₁-C₆)- alkyle ;
où, pour le radical R21, les significations phényle non substitué et benzyle non substitué, sont exclues ;
R3 signifie, indépendamment, (C₂-C₁₀)-alcényle ou (C₁- C₆)-alkylène-(C₆₋C₁₀)-aryle, où les radicaux alcényle et (C₁-C₆)-alkylène-(C₆-C₁₀)-aryle peuvent être monosubstitués ou polysubstitués par F, Cl, Br ;
R4, R5 signifient H ;
n vaut 0 ;
ainsi que leurs sels physiologiquement acceptables.

5. Composés selon l'une ou plusieurs des revendications 1 à 4 destinés à être utilisés comme médicament.

6. Médicament contenant un ou plusieurs des composés selon l'une ou plusieurs des revendications 1 à 4.

7. Médicament contenant un ou plusieurs des composés selon l'une ou plusieurs des revendications 1 à 4 et au moins une autre substance active.

8. Médicament selon la revendication 7, **caractérisé en ce qu'**il contient comme autre substance active un(e) ou plusieurs antidiabétiques, substances actives hypoglycémiques, inhibiteurs de HMGCoA-réductases, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène synthétase, antagonistes de la lipoprotéine (a), inhibiteurs de lipase, insulines, sulfonylurées, biguanides, méglitinides, thiazolidinediones, inhibiteurs d'α-glucosidase, substances actives agissant sur le canal potassique dépendant de l'ATP des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, antagonistes du récepteur de CB1, agonistes de la MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, hormones de croissance, composés libérant les hormones de croissance, agonistes de TRH, modulateurs 2 ou 3 de la protéine découplante, agonistes de la leptine, agonistes de DA (bromocriptine, doprexine), inhibiteurs de lipase/amylase, modulateurs de PPAR, modulateurs de RXR ou agonistes de TR-β ou amphétamines.

9. Utilisation des composés selon l'une ou plusieurs des revendications 1 à 4 pour la préparation d'un médicament destiné à abaisser la glycémie.

10. Utilisation des composés selon l'une ou plusieurs des revendications 1 à 4 pour la préparation d'un médicament destiné au traitement du diabète de type II.

11. Utilisation des composés selon l'une ou plusieurs des revendications 1 à 4 pour la préparation d'un médicament destiné au traitement des troubles du métabolisme lipidique et des hydrates de carbone.

12. Utilisation des composés selon l'une ou plusieurs des revendications 1 à 4 pour la préparation d'un médicament destiné au traitement de phénomènes artérioscléreux.

13. Utilisation des composés selon l'une ou plusieurs des revendications 1 à 4 pour la préparation d'un médicament destiné au traitement de la résistance à l'insuline.

14. Procédé pour la préparation d'un médicament contenant un ou plusieurs des composés selon l'une ou plusieurs des revendications 1 à 4, **caractérisé en ce que** la substance active est mélangée avec un support pharmaceutiquement approprié et ce mélange est amené dans une forme appropriée pour l'administration.
